(19) Europäisches Patentamt European Patent Office Office européen des brevets

(11) **EP 3 250 189 B1**

(12) **EUROPEAN PATENT SPECIFICATION**

(45) Date of publication and mention of the grant of the patent:
**21.08.2019  Bulletin 2019/34**

(21) Application number: **15756498.0**

(22) Date of filing: **31.07.2015**

(51) Int Cl.:
***A61K 9/20*** (2006.01)

(86) International application number:
**PCT/UA2015/000067**

(87) International publication number:
**WO 2016/122432 (04.08.2016 Gazette 2016/31)**

(54) **ENTEROSORBENT**

ENTEROSORBENS

ENTÉROSORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority:  **27.01.2015  UA 201500641**

(43) Date of publication of application:
**06.12.2017   Bulletin 2017/49**

(73) Proprietors:
  • **Boiko, Olexandr Mikhailovich**
    **Kyiv 01103 (UA)**
  • **Kurchenko, Oleg Volodimirovich**
    **Kyiv 04053 (UA)**

(72) Inventors:
  • **Boiko, Olexandr Mikhailovich**
    **Kyiv 01103 (UA)**
  • **Kurchenko, Oleg Volodimirovich**
    **Kyiv 04053 (UA)**

(74) Representative: **Benatov, Samuil Gabriel**
    **Dr. EMIL BENATOV & PARTNERS**
    **6, Asen Peykov Str.**
    **1113 Sofia (BG)**

(56) References cited:
    **RU-C2- 2 180 577**

  • **Kurchenko Oleh Volodymyrovych; Boiko Oleksandr Mykhailovych: "UA85371U - Enterosorbent", , 11 November 2013 (2013-11-11), pages 1-4, XP055224197, Retrieved from the Internet: URL:http://uapatents.com/4-85371-enterosor bent.html [retrieved on 2015-10-28] cited in the application**

**Description**

Technical Field

The invention relates to medications for treatment of digestive track or digestive system disorders, more specifically to enterosorbents which can be used as a drug or a dietary supplement.

Background Art

From patent RU 2234931 enterosorbent which contains silicon dioxide and is made in the form of a tablet is known. Besides silicon dioxide this enterosorbent contains lignin hydrolised and microcrystalline cellulose which also have adsorptive capacity as well as excipients which serve to obtain the tablet: pectin, starch and calcium stearate or magnesium stearate.

The disadvantage of this enterosorbent is the presence of lignin hydrolised which can cause dyspeptic events, constipation and allergic reactions.

Also, known is (utility model patent UA 41849) enterosorbent in the form of a tablet with the following composition of components, wt. %:

| | |
|---|---|
| silicon dioxide | 20 - 40 |
| microcrystalline cellulose | 15 - 20 |
| starch | 3 - 5 |
| sugar | 30 - 40 |
| croscarmellose sodium | 15 - 20 |

The absence of lignin hydrolised in this enterosorbent and the use of components in the stated proportions provide its high adsorption capacity, including protein-binding activity. At the same time this enterosorbent does not cause side effects in the form of dyspeptic events, constipation and allergic reactions, which allows, in particular, to use it as a dietary supplement.

However, the process of making this enterosorbent due to the use of sugar in it, which serves for preparing a wetting agent in manufacturing tablets, takes a long time, is energy intensive, and the enterosorbent itself does not quite easily decompose and thus the effect of the drug in the body is delayed.

Therefore, the task was to develop enterosorbent in the form of a tablet that easily decomposes in the body, has a high sorption capacity and the process of the manufacture of which does not take much time and is not energy intensive.

In enterosorbent according to utility model patent UA 85371 this task is solved by the fact that instead of sugar dextrose (Roferose®) is used, and the components are taken in the following ratio, wt. %:

| | |
|---|---|
| silicon dioxide colloidal | 27,00-33,00 |
| microcrystalline cellulose | 26,74-32,68 |
| dextrose (Roferose®) | 20,19-24,67 |
| croscarmellose sodium | 12,47-15,25 |
| pharmacopeial talc | 2,70-3,30 |
| magnesium stearate | 0,90-1,10. |

According to this patent tablets of white color with smooth surface are obtained. However, it was observed that upon long-term storage (about two and a half months) at high temperature (about 30°C) and humidity (about 60%) the color of tablets may change to light yellow, with further storage under the same conditions becomes light brown. In this case the consistency of the tablet becomes more friable.

Summary of Invention

The object of the invention is to develop enterosorbent in the form of a tablet that during storage and transportation does not change its color and consistency.

In enterosorbent in the form of a tablet containing from 25 to 35 wt.% silicon dioxide colloidal, 15 to 33 wt.% microcrystalline cellulose, with the balance being excipients which include a substance which serves for preparing a wetting agent in manufacturing tablets, the objective is achieved by the fact that as the excipient it contains sorbitol in amount of 20 to 30% by weight of the tablet.

Preferably, the components in the table are in the following proportions, wt/ %:

| silicon dioxide colloidal | 25,00 - 35,00 |
|---|---|
| microcrystalline cellulose | 15,00 - 33,00 |
| sorbitol | 20,00 - 30,00 |
| croscarmellose sodium | 12,00 - 16,00 |
| potato starch | 2,00 - 6,00 |
| magnesium stearate | 0,50 - 1,10. |

When the content of at least some components is beyond the specified ranges, the objective is not fully achieved.

Preferably, the components in the tablet having mass 700 mg are in the following amounts, mg:

| silicon dioxide colloidal | 210,00 |
|---|---|
| microcrystalline cellulose | 185,46 |
| sorbitol | 175,00 |
| croscarmellose sodium | 97,34 |
| potato starch | 28,00 |
| magnesium stearate | 4,20. |

The technical result attained by the invention is that the tablets even in extreme conditions of storage and transportation (high temperature and humidity) do not change their color and consistency. In addition, tablets are softer and easier to chew, i.e. their organoleptic properties are improved.

Another technical result achieved by the invention is that the enterosorbent can safely be used by patients with diabetes because sorbitol, despite the sweet taste, is not a carbohydrate. The use of sorbitol in the composition of the enterosorbent contributes to the saving of the body's expense of B group vitamins - pyridoxine, thiamine, biotin, and promotes the growth of intestinal flora that synthesizes these vitamins.

Unexpected technical result achieved by the invention is the enterosorbent sorption capacity improvement by 10 - 15%.

Best Mode for Carrying out the Invention

The tablets according to the invention were prepared in the following manner.

First, a wetting agent was prepared. In a container with a stirrer 22,80 kg of purified water was poured, heated to 60°C and at operating mixer batchwise loaded 10,50 kg of sorbitol and continued stirring at 70-80°C until sorbitol fully dissolved and clear solution formed. To obtain the second portion of the wetting agent the operation was repeated. After that, the operation of obtaining wet granulate was carried out in the highspeed mixer-granulator VHG. 1.68 kg of starch and 12,60 kg of silicon dioxide colloidal were loaded in the mixer-granulator and mixing was carried out for 2-3 min at the impeller rotation speed of 20-30 rpm. Not stopping mixing, one portion of the wetting agent was loaded through a hopper and the process of wetting and granulating mass was carried out. The granulate was discharged into prearranged containers. To obtain the second portion of the wet granulate the operation was repeated.

The operation of drying the wet granulate was carried out by means of the fluid bed granulator VFG - 120B. Thereto, the fluid bed granulator was preheated at the inlet air temperature of 60±5°C for 15-20 minutes, then it was loaded with a portion of the wet granulate, and the granulate was dried at the rotation velocity of 200-500 rpm to the residual moisture of 1,0-3,0%. The same operation was carried out with the second portion of the wet granulate. Then, by means of the calibrator VFZ-300 with the sieve size of 1 mm the dry granulate of the two portions was calibrated into a pre-calibrated tank Farbamin 3-8.1.

Further, the operation of dusting was performed. Thereto, the tank Farbamin 3-8.1 with the dry granulate was connected to the mixing system Farbamin VZD-400IBC, and dusting components - 22.25 kg microcrystalline cellulose, 0.528 kg magnesium stearate and 11,68 kg sodium croscarmelose were loaded into the system. Dusting was carried out at the rotation velocity of 4-8 rpm for 5-7 min.

As a result of all operations approximately 84 kg mass for tableting was received.

The process of tableting was performed using the tabletpress Pharmapress 250, where the diameter of the punches was 12.0 mm and the radius of the sphere was 1.1 D. The receiving hopper of the tabletpress from the tank Farbamin C-8.1 was filled with the mass for tableting, and with the use of 0.528 kg magnesium stearate tableting was implemented. The force of the main pressing was maintained within the range 50-59 kN.

120000 tablets having mass 700 mg were made with the following composition of components, mg:

| | |
|---|---|
| silicon dioxide colloidal | 210,00 |
| microcrystalline cellulose | 185,46 |
| sorbitol | 175,00 |
| croscarmellose sodium | 97,34 |
| potato starch | 28,00 |
| magnesium stearate | 4,20 |

The tablets had a white color and a smooth surface. On the basis of the experts' evidence it has been established that these tablets are softer and easier to chew than the tablets according to utility model patent UA 85371, and during storage for two months at a temperature of about 30°C and humidity about 60% their color did not change.

The comparative tests of the enterosorbent obtained and the enterosorbent according to utility model patent UA 85371 were carried out.

The tests were performed by absorption spectrophotometry (ГФу, 2.2.25).

Gelatine solution (a). 60 ml of water was added to 600 mg of gelatine and left for 1 hour. The swollen gelatine was dissolved with stirring and at the temperature from 50°C to 60°C, cooled, the solution volume was brought up to 100 ml by means of water. The obtained solution was suitable for use within 4 hours.

Gelatine solution (b). 5,0 ml of the gelatine solution (a) was brought up to 25.0 ml by means of biuretic reagent and stirred. The shelf life of the solution was 7 days.

The test solution. About 0.4 g of the carefully powdered drug was placed in a conical flask with a capacity of 100 ml, 25.0 ml of the gelatine solution was added and shaken for 30 min in a shaking apparatus, then centrifuged for 20 min at the velocity of 3000 rpm.

5.0 ml of the upper transparent layer was placed in a volumetric flask with the capacity of 25.0 ml and by means of the biuretic reagent the solution volume was brought up to the mark and stirred.

In 30 minutes the optical density of the test solution and the gelatine solution (b) was measured by means of a spectrophotometer at a wavelength of 560 nm using the biuretic reagent as a compensation solution.

The sorption capacity of the drug (X), in mg/g, was determined by the formula:

$$X = \frac{(A_0 - A_1) \cdot m_0 \cdot 25 \cdot 100}{A_0 \cdot m_1 \cdot 100 \cdot (100 - W)} = \frac{(A_0 - A_1) \cdot m_0 \cdot 25}{A_0 \cdot m_1 \cdot (100 - W)},$$

where:

$A_0$    - the optical density of the gelatine solution b);
$A_1$    - the optical density of the test solution;
$m_0$    - gelatine sample weight taken for prepating the gelatine solution a), in milligrams;
$m_1$    - the drug sample weight, in grams;
$W$    - weight loss on drying, per cent.

The sorption capacity of the enterosorbent according to UA 85371 was within 330 - 340 mg/g, the sorption capacity the enterosorbent according to the invention was within 374-390 mg/g. That is, the sorption capacity of the enterosorbent increased by 10 -15%.

## Claims

1.  Enterosorbent in the form of a tablet containing 25 - 35 wt. % of silicon dioxide colloidal, 15 to 33 wt. % of microcrystalline cellulose, with the balance being excipients, **characterized in that** the enterosorbent comprises sorbitol in amount of 20 to 30% by weight of the tablet, as an excipient serving to prepare a wetting agent by the manufacture of the tablet.

2.  The enterosorbent according to claim 1, wherein the components in a tablet are in the following proportions, wt/ %:

| silicon dioxide colloidal | 25,00 - 35,00 |
|---|---|
| microcrystalline cellulose | 15,00 - 33,00 |
| sorbitol | 20,00 - 30,00 |
| croscarmellose sodium | 12,00 - 16,00 |
| potato starch | 2,00 - 6,00 |
| magnesium stearate | 0,50 - 1,10. |

3. The enterosorbent according to claim 1 or 2, wherein the components in a tablet are in the following amounts, mg:

| silicon dioxide colloidal | 210,00 |
|---|---|
| microcrystalline cellulose | 185,46 |
| sorbitol | 175,00 |
| croscarmellose sodium | 97,34 |
| potato starch | 28,00 |
| magnesium stearate | 4,20 |

**Patentansprüche**

1. Enterosorbens in Tablettenform mit 25 - 35 Gew .-% kolloidales Siliciumdioxid und 15 bis 33 Gew .-% mikrokristalline Cellulose, den Rest bilden Hilfsstoffe, **dadurch gekennzeichnet, dass** Enterosorbens Sorbit in einer Menge von 20 bis 30 Gew .-% der Tablette als Hilfsstoff enthält, der zur Netzmittelherstellung bei der Tablettenherstellung dient.

2. Enterosorbens nach Anspruch 1, **dadurch gekennzeichnet, dass** die Komponenten in einer Tablette in folgenden Anteilen enthalten sind, Gew .-%,:

| kolloidales Siliciumdioxid | 25,00 - 35,00 |
|---|---|
| mikrokristalline Cellulose | 15,00 - 33,00 |
| Sorbit | 20,00 - 30,00 |
| Croscarmellose-Natrium | 12,00 - 16,00 |
| Kartoffelstärke | 2,00 - 6,00 |
| Magnesiumstearat | 0,50 - 1,10. |

3. Enterosorbens nach Anspruch 1 oder 2, **dadurch gekennzeichnet, dass** die Komponenten in einer Tablette in folgenden Mengen enthalten sind, mg:

| kolloidales Siliciumdioxid | 210,00 |
|---|---|
| mikrokristalline Cellulose | 185,46 |
| Sorbit | 175,00 |
| Croscarmellose-Natrium | 97,34 |
| Kartoffelstärke | 28,00 |
| Magnesiumstearat | 4,20. |

**Revendications**

1. Entérosorbant sous forme d'un comprimé contenant en poids de 25 - 35 % de dioxyde de silicium colloïdal, 15 - 33 % de cellulose microcristalline, le restant étant des excipients, **caractérisé en ce que** l'entérosorbant comprend du sorbitol en quantité de 20 - 30 % en poids du comprimé, comme excipient de préparation d'un humidificateur pour la fabrication des comprimés.

2. Entérosorbant selon la revendication 1, dans lequel les composants du comprimé sont dans les proportions suivantes en poids du comprimé :

| | |
|---|---|
| dioxyde de silicium colloïdal | 25,00 - 35,00 |
| cellulose microcristalline | 15,00 - 33,00 |
| sorbitol | 20,00 - 30,00 |
| croscarmellose sodique | 12,00 - 16,00 |
| amidon de pommes de terre | 2,00 - 6,00 |
| stéarate de magnésium | 0,50 - 1,10. |

3. Entérosorbant selon les revendications 1 ou 2, dans lequel les composants du comprimé sont en quantités suivantes exprimées en mg :

| | |
|---|---|
| dioxyde de silicium colloïdale | 210,00 |
| cellulose microcristalline | 185,46 |
| sorbitol | 175,00 |
| croscarmellose sodique | 97,34 |
| amidon de pommes de terre | 28,00 |
| stéarate de magnésium | 4,20 |

**EP 3 250 189 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RU 2234931
- UA 41849
- UA 85371